# EUROPEAN PATENT SPECIFICATION

(11) **EP 3 669 909 B1**
(45) Date of publication and mention of the grant of the patent: **23.08.2023**
(21) Application number: 20157248.4
(22) Date of filing: 31.10.2016
(51) Int. Cl.: A61M 5/168, A61M 39/08, A61M 39/22, A61M 39/28, A61M 5/14

(54) **MEDICAL LIQUID ADMINISTRATION DEVICE**
MEDIZINISCHE FLÜSSIGKEITSVERABREICHUNGSVORRICHTUNG
DISPOSITIF D'ADMINISTRATION DE LIQUIDE MÉDICAL

(30) Priority: 10.12.2015 JP 2015240691
(43) Date of publication of application: 24.06.2020
(62) Divisional of application: 16872587.7
(73) Proprietor: Nipro Corporation, Osaka-shi, Osaka 531-8510 (JP)
(72) Inventor: SAKAMOTO, Shingo, Kita-ku, Osaka-shi Osaka 531-8510 (JP)
(74) Representative: Germain Maureau

(56) References cited:
- EP-A1- 0 783 895
- EP-A2- 1 129 740
- EP-B1- 1 129 740
- WO-A1-96/09845
- JP-B1- 3 073 979
- US-A- 5 785 681

## Description

### TECHNICAL FIELD

The present disclosure relates to drug solution administration devices and, in particular, to a drug solution administration device allowing a patient to control bolus administration of a drug solution.

### BACKGROUND ART

There are drug solution administration techniques to continuously inject a drug solution into a patient for, for example, administering an analgesic to a patient after surgery and an anticancer drug to a cancer patient. Continuous administration usually involves administering a drug solution at a flow rate ranging from approximately 0.5 mL/h to 10 mL/h for about half a day to several days.

In such continuous administration, a dosage of the drug solution is basically constant. In some cases, however, a medical practitioner intends to provide bolus administration with the dosage of the drug solution temporarily increased. For example, when an analgesic is administered after surgery, a dosage of the analgesic can be temporarily increased, depending on severity of the pain that the patient feels. Such bolus administration is provided under supervision of a medical practitioner. In recent years, however, patient controlled analgesia (PCA) has been provided by patients themselves within a limited extent.

When a patient himself or herself provides the bolus administration of a drug solution, required is a drug solution administration device which is easy to operate and equipped with an adequate safety mechanism to prevent drug solution overdose. Drug solution administration devices capable of the bolus administration are classified into two types: one utilizes a precision infusion pump capable of controlling a dispensing rate of a drug solution, and the other is a combination of a balloon pump using contraction of a balloon and a drug solution supply line including a flow rate adjuster. The drug solution administration device using a balloon pump has an advantage in easy operation. Moreover, this device requires no power source, making the device portable.

As an example of techniques to temporarily change a dosage with the drug solution administration device using a balloon pump, a bolus administration unit including a sub reservoir reserving a certain amount of drug solution is connected to a drug solution supply line (see Patent Document 1, for example). The drug solution reserved in the sub reservoir is dispensed to the drug solution supply line so that the dosage can be temporarily increased. If the sub reservoir that has already dispensed the drug solution is to gradually reserve the drug solution again through the drug solution supply line, a lockout time can be provided to prevent continuous injection of the drug solution, keeping the drug solution from overdose.

### CITATION LIST

### PATENT DOCUMENT

PATENT DOCUMENT 1: Japanese Unexamined Patent Publication No. H10-99432

It is known from document WO96/09845 a burette apparatus having a delivery tube connecting a fluid bag a burette assembly, via a fluid entry spigot formed in an end cap assembly of the burette assembly, the end cap assembly having a bypass spigot connected to the delivery tube and usually closed by clip, to allow an override for adding liquid to the burette. The end cap assembly closes the upper end of a transparent, cylindrical burette body, into which extends a stainless steel tube extension of the fluid entry spigot, the lower end of the burette body being closed by a lower closure assembly having a check valve and a drip chamber. An outlet tube passes from the drip chamber to the patient via regulating clamp. A float assembly is located in the burette body including an extension rod provided with a resilient cup adapted to engage the open end of the stainless steel tube extension.

It is also known from document EP0783895 a portable analgesic device which comprises a chemical feeder for temporarily storing a predetermined amount of chemical and then discharging the chemical by itself, a first three-way connector connected to an outflow port of the chemical feeder, an intermittent chemical injecting line connected to one outlet of the first three-way connector, a continuous chemical injecting line connected to the other outlet of the first three-way connector, and a second three-way connector for establishing communication between outflow ports of both lines.

Moreover, the document EP1129740 depicts an infusion rate adjusting device for drug solution injectors comprises a tubular flow control unit including plural check valves of different opening pressures, and means for selecting one of said check valves to adjust an infusion rate of the drug solution. The infusion rate adjusting device is simple in operation to set out an infusion rate of the drug solution and is considerably reduced in the production cost.

### SUMMARY

### TECHNICAL PROBLEM

The typical technique to provide a sub reservoir has a problem of extra work required for priming before use. For example, the drug solution administration line is primed with the balloon pump. The bolus administration unit is separately primed with the drug solution injected into the unit, using, for example, a syringe. Then, the bolus administration unit and the drug solution administration line are connected together. Hence, the typical technique requires such troublesome tasks as attaching and removing a connector and injecting the drug solution using a syringe. A port having a check valve could be provided to eliminate the need for attaching and removing the connector. However, providing the port also requires the infusion of the drug solution with a syringe. Moreover, providing the port increases costs of the drug solution administration device.

In view of the forgoing background, it is an object of the present disclosure to implement a drug solution administration device which allows a lockout time to be set to prohibit bolus administration and is easily primed.

### SOLUTION TO THE PROBLEM

The invention is defined by the subject-matter of claim

### 1. Further embodiments are defined in the dependent claims.

An aspect of a drug solution administration device includes: a bolus administration unit providing a drug solution, the bolus administration unit branching off a main flow passage including a flow rate controller controlling a flow rate of the drug solution, wherein the bolus administration unit includes: an inflow passage connected to the main flow passage on a proximal end side of the flow rate controller; an outflow passage connected to the main flow passage on a distal end side of the flow rate controller; a reservoir connected between the inflow passage and the outflow passage, and reserving the drug solution; and a control portion controlling the reserve of the drug solution in the reservoir and dispensing of the drug solution from the reservoir, the inflow passage includes: a first flow passage; and a second flow passage smaller in flow passage cross-sectional area than the first flow passage, and the control portion includes: a first closer operable to close the first flow passage; and a second closer operable to open and close the outflow passage..

In another aspect of the drug solution administration device, the bolus administration unit may operate in: a priming mode in which the bolus administration unit is filled with the drug solution with the first closer and the second closer opened; a reservation mode in which the drug solution is reserved in the reservoir with the first closer and the second closer closed; and a bolus administration mode in which the drug solution reserved in the reservoir is dispensed with the first closer kept closed and the second closer opened, and the control portion may control a change from the priming mode to the reservation mode, and a change between the reservation mode and the bolus administration mode.

In another aspect of the drug solution administration device, the control portion may include: a first operation button causing, when operated, the bolus administration unit to change from the priming mode to the reservation mode, and locked once operated, a second operation button causing, when operated, the bolus administration unit to change from the reservation mode to the bolus administration mode, and after the drug solution reserved in the reservoir is dispensed, the second operation button may automatically return and cause the bolus administration unit to change from the bolus administration mode to the reservation mode.

In another aspect of the drug solution administration device, the main flow passage may include a pump dispensing the drug solution, and in the priming mode, the drug solution to fill the inflow passage, the reservoir, and the outflow passage is dispensed from the pump.

### ADVANTAGES OF THE INVENTION

The drug solution administration device of the present disclosure allows a lockout time to be set to prohibit bolus administration, and is easily primed.

### BRIEF DESCRIPTION OF THE DRAWINGS

[FIG. 1] FIG. 1 is a schematic view illustrating a drug solution administration device according to an embodiment.
[FIG. 2] FIG. 2 is a schematic view illustrating a general configuration of a bolus administration unit body.
[FIG. 3] FIG. 3 is a perspective view illustrating the bolus administration unit body.
[FIG. 4] FIG. 4 is a cross-sectional view taken from line IV-IV of FIG. 3.
[FIG. 5] FIG. 5 is a cross-sectional view illustrating how a first operation button is locked.
[FIG. 6] FIG. 6 is a cross-sectional view of the drug solution administration device according to a modification, the cross-sectional view illustrating how the first operation button is unlocked.
[FIG. 7] FIG. 7 is a cross-sectional view of the drug solution administration device according to the modification, the cross-sectional view illustrating how the first operation button is locked.

### DESCRIPTION OF EMBODIMENT

In the present disclosure, when an appliance is used, an end of the appliance toward a patient is referred to as "distal end", and an end opposite the distal end is referred to as "proximal end."

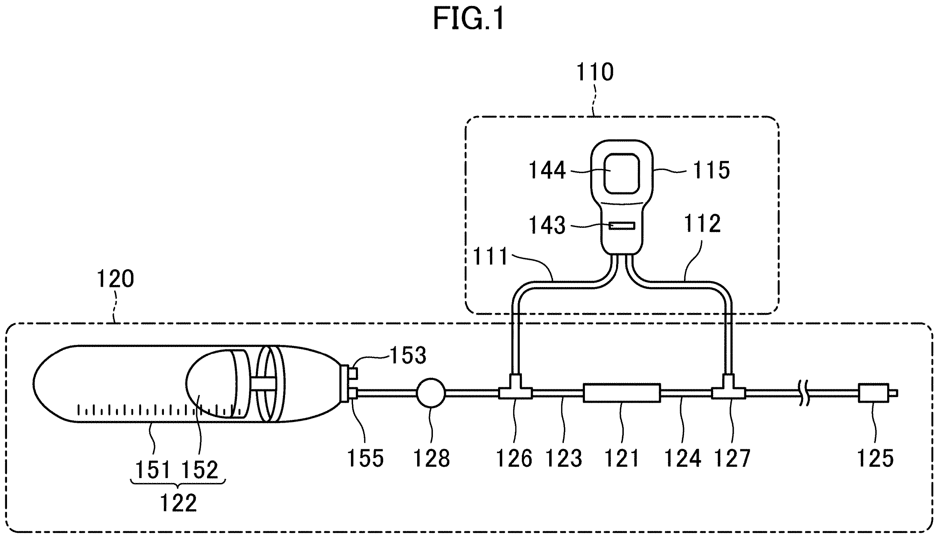

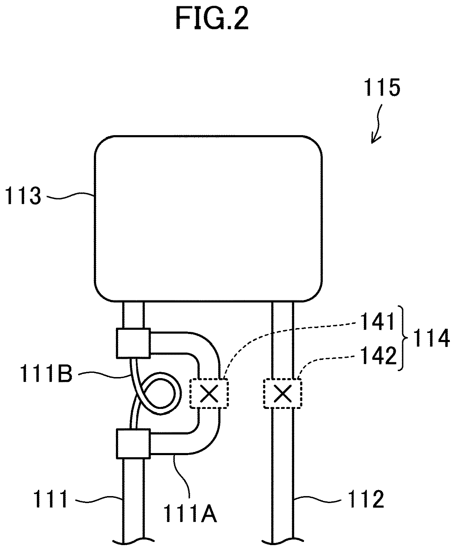

As illustrated in FIGS. 1 and 2, a drug solution administration device of this embodiment includes a bolus administration unit 110 branching off a main flow passage 120 administering a drug solution, and providing bolus administration of the drug solution. The bolus administration unit 110 includes: an inflow passage 111 connected to the main flow passage 120 on a proximal end side of a flow rate controller 121 provided to the main flow passage 120; an outflow passage 112 connected to the main flow passage 120 on a distal end side of the flow rate controller 121; and a bolus administration unit body 115.

The bolus administration unit body 115 includes: a reservoir 113 connected between the inflow passage 111 and the outflow passage 112, and reserving the drug solution; and a control portion 114 controlling the reserve of the drug solution in the reservoir 113 and of the drug solution from the reservoir 113. The inflow passage 111 includes: a first flow passage 111A; and a second flow passage 111B smaller in flow passage cross-sectional area than the first flow passage 111A. The control portion 114 includes: a first closer 141 operable to close the first flow passage 111A; and a second closer 142 operable to open and close the outflow passage 112.

The reservoir 113 can act as a resilient container capable of changing in shape when pressed. For example, the reservoir 113 may be a pouch container formed of such a flexible resin as polyethylene, polypropylene, or polyester, or such a rubber elastic material as polyisoprene or silicone rubber. The reservoir 113 may reserve the drug solution to be administered for a single dosage in the bolus administration. An example of the reserve of the drug solution approximately ranges from 0.5 mL to 10 mL.

The inflow passage 111 and the outflow passage 112 may be flexible tubes. The tubes may be made of such materials as polyvinyl chloride, polyester, polyamide, polyeurethane, polyacrylate, polymethacrylate, or polycarbonate.

The inflow passage 111 has a part in which the inflow passage 111 branches into the first flow passage 111A and the second flow passage 111B. The first flow passage 111A is a regular flexible tube, and functions as a bypass route for quickly priming the bolus administration unit 110. The second flow passage 111B is an orifice tube smaller in flow passage cross-sectional area than the first flow passage 111A. The second flow passage 111B functions as a flow rate controller controlling an amount of the drug solution flowing into the reservoir 113. Use of the first flow passage 111A allows the bolus administration unit 110 to be quickly primed. Meanwhile, the second flow passage 111B can adjust the flow rate of the drug solution flowing into the reservoir 113, successfully adjusting a time period until the drug solution is reserved in the reservoir 113. Such features make it possible to set a lockout time to prohibit bolus administration.

The second flow passage 111B has a passage resistance set to make it possible to adjust the flow rate of the drug solution flowing into the reservoir 113. The passage resistance may be set with a change in an inner diameter and a length of the passage. The inner diameter and the length of the second flow passage 111B may be determined in accordance with a target flow rate. In view of reducing the risk that air in the drug solution is caught on the wall of the passage, followed by the obstruction of the flow of the drug solution, the inner diameter may preferably be approximately 10 µm or larger, and may more preferably be approximately 50 µm or larger. In view of controlling the flow rate, the inner diameter may preferably be 500 µm or smaller, and may more preferably be 300 µm or smaller. In view of controlling the flow rate, the length may preferably be 1 cm or longer. Moreover, in view of controlling the flow rate, the second flow passage 111B is preferably less likely to be deformed, and preferably has an outer diameter approximately 5 times to 500 times larger than the inner diameter.

The bolus administration unit 110 of this embodiment in the above configuration operates in: a priming mode in which the bolus administration unit 110 is filled with the drug solution with the first closer 141 and the second closer 142 opened; a reservation mode in which the drug solution is reserved in the reservoir 113 with the first closer 141 and the second closer 142 closed; and a bolus administration mode in which the drug solution reserved in the reservoir 113 is dispensed with the first closer 141 kept closed and the second closer 142 opened. A change from the priming mode to the reservation mode, and a change between the reservation mode and the bolus administration mode can be controlled by the control portion 114 including a first operation button 143 and a second operation button 144.

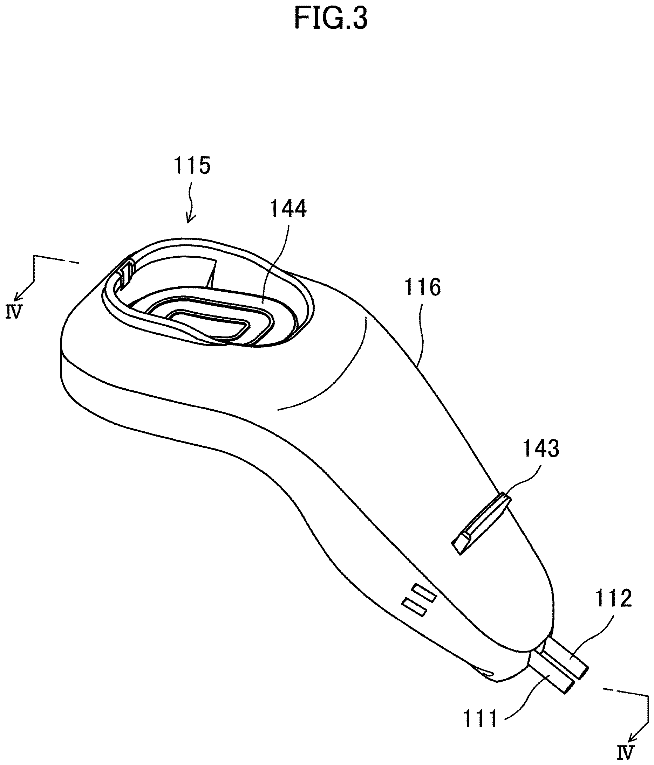

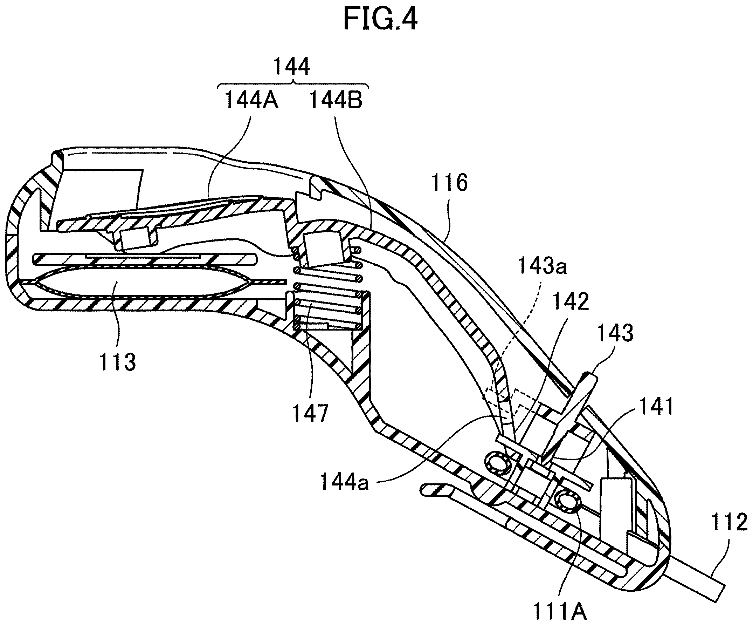

Described below is a specific example of the bolus administration unit body 115 including the control portion 114. As illustrated in FIGS. 3 to 5, the bolus administration unit body 115 of this embodiment includes a housing 116 containing: the reservoir 113; and the control portion 114 controlling the inflow and dispensing of the drug solution into and out of the reservoir 113. The control portion 114 includes: the first operation button 143 and the second operation button 144 both can be operable from the outside of the housing 116; the first closer 141 operated with the first operation button 143; and the second closer 142 operated with the second operation button 144. When used, the bolus administration unit body 115 of this embodiment may be provided in any given orientation. In the description below, the bolus administration unit body 115 is oriented so that the first operation button 143 and the second operation button 144 face upward.

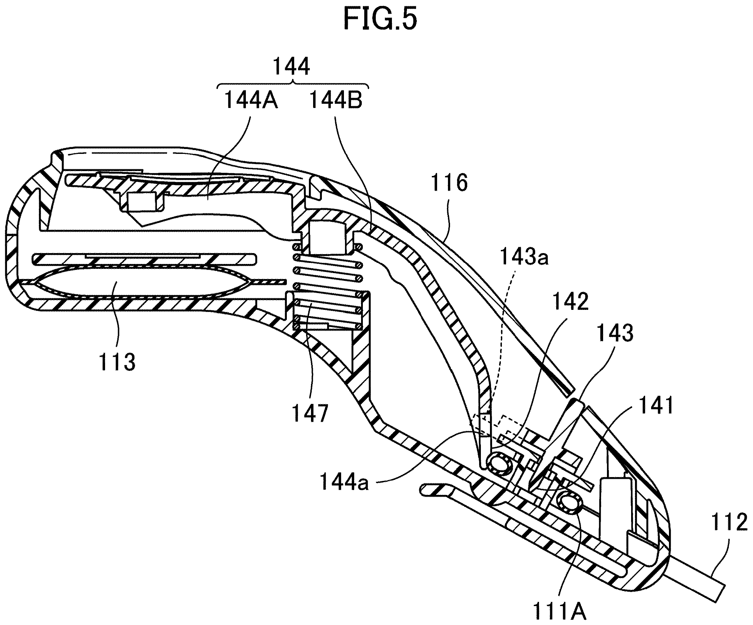

In this embodiment, the first closer 141 is a pressing unit integrally formed with the first operation button 143 and shaped into a plate. As illustrated in FIG. 5, when the first operation button 143 is pressed to press down the pressing unit, the pressing unit moves toward the first flow passage 111A. Hence, the pressing unit can press and close the flexible tube acting as the first flow passage 111A. Once operated, the first operation button 143 is locked in a position in which the pressing unit acting as the first closer 141 presses the first flow passage 111A.

The second closer 142 acting as the pressing unit is formed integrally with the second operation button 144. The second closer 142 can press and close a flexible tube acting as the outflow passage 112. In this embodiment, the second operation button 144 is the pressing unit including: an arm 144B; and a button body 144A provided to a first end of the arm 144B. The arm 144B has a second end acting as the second closer 142. The arm 144B has a center portion supported by a spring 147. When the button body 144A provided to the first end is pressed down, the second closer 142 provided to the second end is lifted. Moreover, the first end is provided above the reservoir 113. When the button body 144A is pressed down, the reservoir 113 is pressed.

The second operation button 144 engages with the first operation button 143 toward the second end. Specifically, the arm 114B is provided with an opening 144a into which an engaging projection 143a, provided to the first operation button 143, is inserted. Hence, as illustrated in FIG. 5, when the first operation button 143 is pressed down and locked, the pressing unit provided to the second end and acting as the second closer 142 also moves toward and closes the outflow passage 112. Simultaneously, the button body 144A provided to the first end of the arm 114B is lifted upward to be operable. When the button body 144A is pressed with the first operation button 143 locked, the second closer 142 provided to the second end is lifted through leverage such that the outflow passage 112 is opened. Simultaneously, the reservoir 113 is pressed such that the drug solution reserved in the reservoir 113 is dispensed from the outflow passage 112 to the main flow passage 120. The inflow passage 111 is shaped into an orifice tube, and thus higher in passage resistance than the outflow passage 112. Thus, most of the drug solution dispensed from the reservoir 113 flows toward the outflow passage 112. Hence, a check valve does not have to be provided to the inflow passage 111. Note that the check valve may be provided to the inflow passage 111.

In this embodiment, the arm 144B of the second operation button 144 is supported by the spring 147. Hence, when a patient stops pressing the button body 144A, the second operation button 144 automatically returns by elasticity of the spring 147. Hence, the reservoir 113 returns to a non-pressed state. Moreover, the second closer 142 acting as a pressing unit moves toward the outflow passage 112 again, and closes the outflow passage 112. Hence, the reservation of the drug solution into the reservoir 113 is resumed.

As described above, when operated by the patient, the first operation button 143 causes the bolus administration unit 110 to change from the priming mode to the reservation mode. Once operated, the first operation button 143 is locked. Furthermore, when operated by the patient, the second operation button 144 causes the bolus administration unit 110 to change from the reservation mode to the bolus administration mode. After the drug solution reserved in the reservoir 113 is dispensed, the second operation button 144 automatically returns and causes the bolus administration unit 110 to change from the bolus administration mode to the reservation mode.

When the second operation button 144 is pressed with no drug solution reserved in the reservoir 113, the second closer 142 is opened; however, the bolus administration is not provided because there is no drug solution to be dispensed. Hence, through controls of a flow rate of the drug solution flowing into the reservoir 113 and a time period until the drug solution is reserved in the reservoir 113, a lockout time can be provided to prohibit the patient from repeatedly taking the bolus administration. Note that provided may be such a device as a lock mechanism to be unlocked when the reservoir 113 bulges in a predetermined size, so that the lock mechanism may keep the second operation button 144 inoperable until the drug solution is sufficiently reserved in the reservoir 113.

The main flow passage 120 includes: a pump 122 reserving and dispensing the drug solution; and the flow rate controller 121 controlling the flow rate of the drug solution flowing through the main flow passage 120. The pump 122 is a balloon pump including a balloon 152 housed in a balloon housing 151. The balloon 152 can let the drug solution flow thereinto from a drug solution inlet 153 and reserve the drug solution under pressure. The balloon 152 can also let the drug solution flow out from a drug solution outlet 155. An internal pressure of the balloon 152 may be set in accordance with, for example, a required flow rate. An example of the internal pressure may range from 20 kPa to 100 kPa. The balloon 152 may be formed of any given material as long as the material is significantly elastic and can create a desired internal pressure. An example of the material includes natural rubber, synthetic rubber, or elastomer.

The flow rate controller 121 controls the flow rate of the drug solution dispensed out of the pump 122 and flowing through the main flow passage 120. The flow rate controller 121 may have any given configuration as long as the flow rate controller 121 can control the flow rate of the drug solution flowing through the main flow passage 120. An example of the configuration may include an orifice tube. The inner diameter and the length of the orifice tube may be determined in accordance with a target flow rate. In view of reducing the risk that air in the drug solution is caught on the wall of the passage, followed by the obstruction of the flow of the drug solution, the inner diameter may preferably be approximately 10 µm or larger, and may more preferably be approximately 50 µm or larger. In view of controlling the flow rate, the inner diameter may preferably be 500 µm or smaller, and may more preferably be 300 µm or smaller. In view of controlling the flow rate, the length may preferably be 1 cm or longer. Moreover, in view of controlling the flow rate, the orifice tube is preferably less likely to be deformed, and preferably has an outer diameter approximately 5 times to 500 times larger than the inner diameter.

The flow rate controller 121 is connected to the drug solution outlet 155 on the proximal end side via a tube 123. The flow rate controller 121 is connected to a connector 125 on the distal end side via a tube 124. The connector 125 can be connected to, for example, a catheter inserted into a blood vessel of a patient. Between the drug solution outlet 155 and the proximal end of the flow rate controller 121, a branch connector 126 is provided to let the inflow passage 111 of the bolus administration unit 110 branches off the main flow passage 120. Between the distal end of the flow rate controller 121 and the connector 125, a branch connector 127 is provided to join the outflow passage 112 of the bolus administration unit 110 to the main flow passage 120.

Between the branch connector 126 and the drug solution outlet 155, a filter 128 is provided. Since the filter 128 is provided in this position, the flow rate controller 121 provided to the main flow passage 120 and the second flow passage 111B provided to the bolus administration unit 110 are less likely to be clogged with such objects as contaminants. Moreover, in this embodiment, the drug solution is not externally injected into the bolus administration unit 110. Hence, a filter does not have to be provided closer toward the distal end than the outflow passage 112 of the bolus administration unit 110 is. Such a feature can reduce the number of parts and an increase in flow passage resistance of the outflow passage 112, contributing to improvement in operability of the bolus administration unit 110. Note that the filter may be provided closer to the distal end than the outflow passage 112 is.

Described below is how the drug solution administration device of this embodiment performs priming. First, the drug solution is injected from the drug solution inlet 153 into the balloon 152. Here, the proximal end of the flow rate controller 121 and the inflow passage 111 of the bolus administration unit 110 can be closed with clamps. When the clamp to the proximal end of the flow rate controller 121 is opened, the drug solution in the balloon 152 is dispensed from the drug solution outlet 155 by the contraction of the balloon 152. Hence, the main flow passage 120 is primed.

After the drug solution is confirmed to flow from the connector 125, the clamp to the proximal end of the flow rate controller 121 is closed and the clamp of the inflow passage 111 is opened. In this state, the bolus administration unit 110 is in the priming mode in which the first closer 141 and the second closer 142 are opened. Hence, the drug solution can run through the first flow passage 111A having a low passage resistance, such that a sufficient flow rate of the drug solution can be maintained. When the reservoir 113 is filled with the drug solution, the drug solution runs through the outflow passage 112 and flows over the connector 125 to the distal end. After the overflow is confirmed, the first operation button 143 of the bolus administration unit 110 is operated so that the first closer 141 and the second closer 142 are closed. As a result, the priming of the main flow passage 120 and the bolus administration unit 110 end, and the bolus administration unit 110 moves to the reservation mode. Hence, the bolus administration unit of this embodiment can quickly perform priming even though no priming port is provided to the inflow passage 111. However, for such cases as an emergency, the inflow passage 111 can be provided with the priming port.

Note that the above example shows that the main flow passage 120 is primed first, followed by the bolus administration unit 110. Alternatively, the bolus administration unit 110 may be primed first, followed by the main flow passage 120.

When the connector 125 of the primed drug solution administration device is connected to, for example, a catheter inserted into a blood vessel of a patient, the drug solution inside the balloon 152 is continuously administered into the blood vessel of the patient by the contraction of the balloon 152. In this case, the dosage of the drug solution is the one set with the flow rate controller 121. If the dosage is to be temporarily increased, the second operation button 144 of the bolus administration unit 110 is pressed. Such an operation opens the second closer 142 and presses the reservoir 113. Hence, the bolus administration unit 110 changes from the reservation mode to the bolus administration mode. The drug solution inside the reservoir 113 is dispensed through the outflow passage 112 to the main flow passage 120, and administered to the patient. After the bolus administration is performed, the patient lifts his or her finger off the second operation button 144. The second operation button 144 then automatically returns such that the bolus administration unit 110 changes from the bolus administration mode to the reservation mode. After the change from the bolus administration mode to the reservation mode, it takes a predetermined time until the drug solution is reserved again in the reservoir 113. This time is determined by the flow rate to be set in accordance with the second flow passage 111B. Moreover, once operated, the first operation button 143 is locked so that the first flow passage 111A acting as a bypass cannot be opened. Such a feature allows the first operation button 143 to function as a safety device to keep the bolus administration from overuse.

The drug solution administration device administers the drug solution into a body. Thus, the interior of the flow passage of the drug solution administration device is sterilized. An example of the sterilization typically includes ethylene oxide gas (EOG) sterilization, electron beam sterilization, or γ-ray sterilization. To efficiently perform the EOG sterilization, preferably, the flow passage should be opened and the gas should sufficiently run through the flow passage. In the drug solution administration device of this embodiment, the flow passage does not have to include therein, for example, a check valve to block the flow passage. Such a feature facilitates the EOG sterilization.

In this embodiment, the balloon pump using the contraction of the balloon is introduced as a pump. Alternatively, any given pump may be introduced as long as the drug solution can be dispensed by a predetermined pressure. An example of the pump may be a pump using gas pressure.

In this embodiment, orifice tubes are used as the flow rate controller 121 and the second flow passage 111B. Alternatively, another configuration may be used if a predetermined flow rate is achieved by passage resistance. For example, check valves may be provided instead of the orifice tubes.

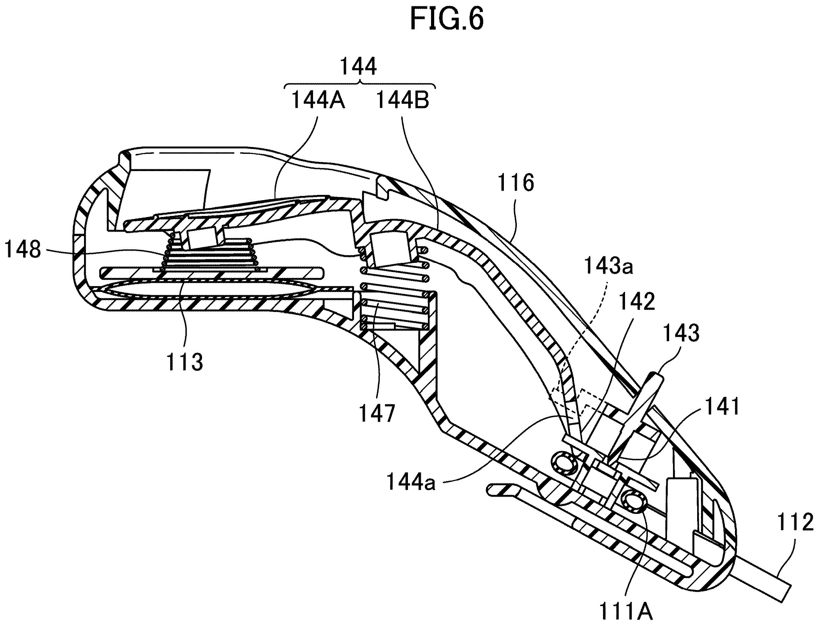

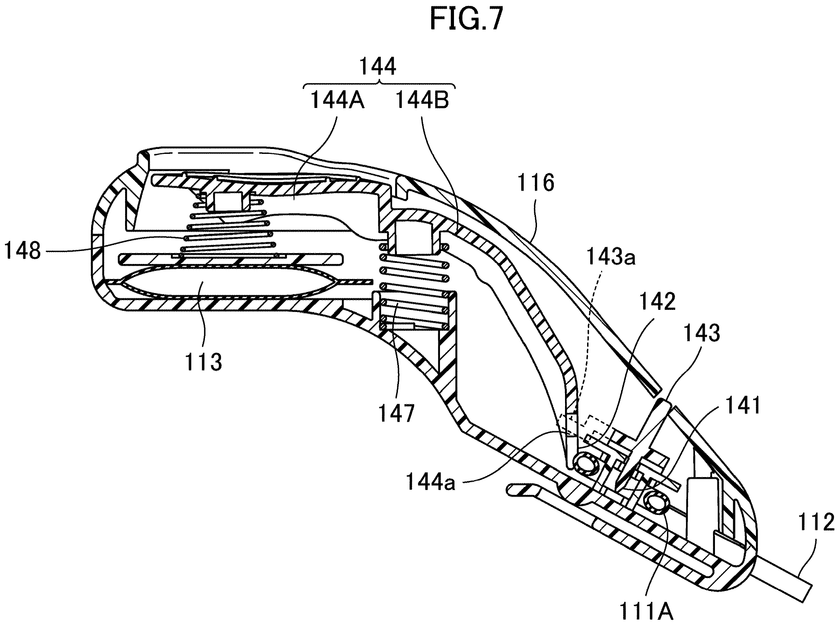

The configuration of the bolus administration unit body 115 described in this embodiment is an example. The bolus administration unit body 115 may have another configuration as long as the first closer 141 and the second closer 142 perform predetermined operations. For example, as illustrated in FIGS. 6 and 7, a spring 148 may be provided between the button body 144A and the balloon 113. When the patient lifts his or her finger off the pressed button body 144A, such a feature allows the button body 144A to easily return to a non-pressed position. Hence, the second closer 142 integrally formed with the second operation button 144 is likely to return to a position in which the second closer 142 presses down, and closes, the flexible tube; namely the outflow passage 112. Note that the FIGS. 6 and 7 illustrate an example that the spring 148 is a conical spring. Alternatively, the spring 148 may have any given configuration as long as the configuration allows the button body 144A to easily return to the non-pressed position.

The drug solution administration device of this embodiment can be used for PCA. Other than that, the medical administration device can be used for, for example, administration of heparin, chemical anesthesia, or anticancer drug.

### INDUSTRIAL APPLICABILITY

The drug solution administration device of the present disclosure allows a lockout time to be set to prohibit bolus administration, and is easily primed. The drug solution administration device is useful for such treatments as PCA.

### DESCRIPTION OF REFERENCE CHARACTERS

- 110: Bolus Administration Unit
- 111: Inflow Passage
- 111A: First Flow Passage
- 111B: Second Flow Passage
- 112: Outflow Passage
- 113: Reservoir
- 114: Control Portion
- 115: Bolus Administration Unit Body
- 116: Housing
- 120: Main Flow Passage
- 121: Flow Rate Controller
- 122: Pump
- 123: Tube
- 124: Tube
- 125: Connector
- 126: Branch Connector
- 127: Branch Connector
- 128: Filter
- 141: First Closer
- 142: Second Closer
- 143: First Operation Button
- 143a: Engaging Projection
- 144: Second Operation Button
- 144A: Button Body
- 144B: Arm
- 144a: Opening
- 147: Spring
- 148: Spring
- 151: Balloon Housing
- 152: Balloon
- 153: drug solution Inlet
- 155: drug solution Outlet

## Claims

1. A bolus administration unit (110) arranged for branching off a flow passage (120) of a drug solution administration device, being **characterized by** comprising:
an inflow passage (111) for connecting to the flow passage (120);
an outflow passage (112) for connecting to the flow passage (120) more distally than the inflow passage (111);
a reservoir (113) connected between the inflow passage (111) and the outflow passage (112), and reserving the drug solution as a reserve of the drug solution; and
a control portion (114) controlling the reserve of the drug solution in the reservoir (113) and dispensing of the drug solution from the reservoir (113),
wherein the inflow passage (111) has a part in which the inflow passage (111) branches into a first flow passage (111A) and a second flow passage (111B) smaller in flow passage cross-sectional area than the first flow passage (111A), and
the control portion (114) includes: a first closer (141) operable to close the first flow passage (111A); and a second closer (142) operable to close the outflow passage (112), linked with the first closer (141) and operable to open and close the outflow passage (112) when the first flow passage (111A) is closed.

2. The bolus administration unit (110) of claim 1, wherein the bolus administration unit (110) operates in: a priming mode in which the bolus administration unit (110) is filled with the drug solution with the first closer (141) and the second closer (142) opened; a reservation mode in which the drug solution is reserved in the reservoir (113) with the first closer (141) and the second closer (142) closed; and a bolus administration mode in which the drug solution reserved in the reservoir (113) is dispensed with the first closer (141) kept closed and the second closer (142) opened, and
the control portion (114) controls a change from the priming mode to the reservation mode, and a change between the reservation mode and the bolus administration mode.

3. The bolus administration unit (110) of claim 2, wherein
the control portion (114) includes:
a first operation button (143) causing, when operated, the bolus administration unit to change from the priming mode to the reservation mode, and locked once operated,
a second operation button (144) causing, when operated, the bolus administration unit (110) to change from the reservation mode to the bolus administration mode, and
after the drug solution reserved in the reservoir (133) is dispensed, the second operation button (144) automatically returns and causes the bolus administration unit (110) to change from the bolus administration mode to the reservation mode.

## Patentansprüche

1. Bolus-Verabreichungseinheit (110), die zum Abzweigen eines Strömungsdurchgangs (120) einer Vorrichtung zur Verabreichung einer Medikamentenlösung angeordnet ist, **dadurch gekennzeichnet, dass** sie Folgendes umfasst:
Einen Einströmdurchgang (111) zum Verbinden mit dem Strömungsdurchgang (120);
einen Ausströmdurchgang (112) zum Verbinden mit dem Strömungsdurchgang (120), der weiter entfernt ist als der Einströmdurchgang (111);
ein Reservoir (113), das zwischen dem Einströmdurchgang (111) und dem Ausströmdurchgang (112) verbunden ist und die Medikamentenlösung als eine Reserve der Medikamentenlösung aufbewahrt; und
einen Steuerabschnitt (114), der die Reserve der Medikamentenlösung in dem Reservoir (113) und die Abgabe der Medikamentenlösung aus dem Reservoir (113) steuert,
wobei der Einströmdurchgang (111) einen Teil aufweist, in dem sich der Einströmdurchgang (111) in einen ersten Strömungsdurchgang (111A) und einen zweiten Strömungsdurchgang (111B) verzweigt, der in seinem Strömungsdurchgangsquerschnittsbereich kleiner ist als der erste Strömungsdurchgang (111A), und
der Steuerabschnitt (114) beinhaltet: Einen ersten Schließer (141), der betreibbar ist, um den ersten Strömungsdurchgang (111A) zu schließen; und einen zweiten Schließer (142), der betreibbar ist, um den Ausströmdurchgang (112) zu schließen, der mit dem ersten Schließer (141) verknüpft und betreibbar ist, um den Ausströmdurchgang (112) zu öffnen und zu schließen, wenn der erste Strömungsdurchgang (111A) geschlossen ist.

2. Bolus-Verabreichungseinheit (110) nach Anspruch 1, wobei die Bolus-Verabreichungseinheit (110) betrieben wird in: Einem Ansaugmodus, in dem die Bolus-Verabreichungseinheit (110) mit der Medikamentenlösung gefüllt wird, wobei der erste Schließer (141) und der zweite Schließer (142) geöffnet sind; einen Reservierungsmodus, in dem die Medikamentenlösung in dem Reservoir (113) bei geschlossenem ersten Schließer (141) und geschlossenem zweiten Schließer (142) aufbewahrt wird; und einen Bolusverabreichungsmodus, in dem die in dem Reservoir (113) aufbewahrte Medikamentenlösung bei geschlossen gehaltenem ersten Schließer (141) und geöffnetem zweiten Schließer (142) abgegeben wird, und
der Abschnitt (114) ein Wechseln von dem Ansaugmodus zu dem Reservierungsmodus und ein Wechseln zwischen dem Reservierungsmodus und dem Bolusverabreichungsmodus steuert.

3. Bolus-Verabreichungseinheit (110) nach Anspruch 2, wobei
die Steuereinheit (114) Folgendes beinhaltet:
Eine erste Bedientaste (143), die, wenn sie betrieben wird, die Bolus-Verabreichungseinheit dazu veranlasst, die Verabreichungseinheit vom Ansaugmodus in den Reservierungsmodus zu wechseln, und die, wenn sie einmal betrieben wird, gesperrt ist,
eine zweite Bedientaste (144), die, wenn sie betrieben wird, die Bolus-Verabreichungseinheit (110) dazu veranlasst, vom Reservierungsmodus in den Bolusverabreichungsmodus zu wechseln, und
die zweite Bedientaste (144) automatisch zurückkehrt und bewirkt, dass die Bolus-Verabreichungseinheit (110) vom Bolusverabreichungsmodus in den Reservierungsmodus wechselt, nachdem die in dem Reservoir (133) reservierte Medikamentenlösung abgegeben wurde.

## Revendications

1. Unité d'administration en bolus (110) agencée pour bifurquer d'un passage d'écoulement (120) d'un dispositif d'administration de solution médicamenteuse, **caractérisée en ce qu'**elle comprend :
un passage d'entrée (111) pour se connecter au passage d'écoulement (120) ;
un passage de sortie (112) pour se connecter au passage d'écoulement (120) de manière plus distale que le passage d'entrée (111) ;
un réservoir (113) relié entre le passage d'entrée (111) et le passage de sortie (112), et réservant la solution médicamenteuse comme réserve de la solution médicamenteuse ; et
une partie de commande (114) commandant la réserve de la solution médicamenteuse dans le réservoir (113) et distribuant la solution médicamenteuse depuis le réservoir (113),
dans laquelle le passage d'entrée (111) a une partie dans laquelle le passage d'entrée (111) bifurque en un premier passage d'écoulement (111A) et un deuxième passage d'écoulement (111 B) dont la section transversale de passage d'écoulement est plus petite que celle du premier passage d'écoulement (111A), et
la partie de commande (114) comprend : un premier obturateur (141) pouvant fonctionner pour fermer le premier passage d'écoulement (111A) ; et un deuxième obturateur (142) pouvant fonctionner pour fermer le passage de sortie (112), relié au premier obturateur (141) et pouvant fonctionner pour ouvrir et fermer le passage de sortie (112) lorsque le premier passage d'écoulement (111A) est fermé.

2. Unité d'administration en bolus (110) de la revendication 1, dans laquelle l'unité d'administration en bolus (110) fonctionne dans: un mode d'amorçage dans lequel l'unité d'administration en bolus (110) est remplie de la solution médicamenteuse avec le premier obturateur (141) et le deuxième obturateur (142) ouverts ; un mode de réservation dans lequel la solution médicamenteuse est réservée dans le réservoir (113) avec le premier obturateur (141) et le deuxième obturateur (142) fermés ; et un mode d'administration en bolus dans lequel la solution médicamenteuse réservée dans le réservoir (113) est distribuée avec le premier obturateur (141) maintenu fermé et le deuxième obturateur (142) ouvert, et
la partie de commande (114) commande un changement du mode d'amorçage au mode de réservation, et un changement entre le mode de réservation et le mode d'administration en bolus.

3. Unité d'administration en bolus (110) de la revendication 2, dans laquelle
la partie de commande (114) comprend :
un premier bouton d'actionnement (143) amenant, lorsqu'il est actionné, l'unité d'administration en bolus à passer du mode d'amorçage au mode de réservation, et verrouillé une fois actionné,
un deuxième bouton d'actionnement (144) amène, lorsqu'il est actionné, l'unité d'administration en bolus (110) à passer du mode de réservation au mode d'administration en bolus, et
après la distribution de la solution médicamenteuse réservée dans le réservoir (133), le deuxième bouton d'actionnement (144) revient automatiquement et amène l'unité d'administration en bolus (110) à passer du mode d'administration en bolus au mode de réservation.
